# DEMANDE DE BREVET EUROPEEN

(11) **EP 4 442 344 A1**
(43) Date de publication de la demande: **09.10.2024**
(21) Numéro de dépôt: 24163629.9
(22) Date de dépôt: 14.03.2024
(51) Int. Cl.: B01D 15/18, B01D 11/02, G01N 30/42, G01N 30/58, C07D 311/80, A61K 31/4525, A61K 36/67, C07C 37/82, C07D 295/023

(54) **PROCÉDÉS DE SÉPARATION ET DE PURIFICATION D'UN COMPOSÉ ORGANIQUE CONTENU DANS UNE MATIÈRE D'ORIGINE NATURELLE**

(30) Priorité: 24.03.2023 FR 2302849
(71) Demandeur: Gilson Purification, 56890 Saint-Avé (FR)
(72) Inventeur: AUDO, Grégoire, 56860 SÉNÉ (FR); LOYET, Célestine, 35830 BETTON (FR); BOULHO, Romain, 56610 ARRADON (FR)
(74) Mandataire: Brevalex

(57) **Abrégé**

L'invention se rapporte à un procédé de séparation d'un composé organique contenu dans une matière d'origine naturelle, ledit procédé comprenant au moins une étape d'extraction liquide/liquide au moyen d'un système de solvants biphasique. Ce système de solvants biphasique comprend de l'hexaméthyldisiloxane, de l'éthanol et de l'eau.

L'invention se rapporte également à un procédé de purification d'un tel composé organique mettant en oeuvre ce procédé de séparation.

## Description

### DOMAINE TECHNIQUE DE L'INVENTION

La présente invention se rapporte au domaine de la séparation de composés organiques contenus dans une matière d'origine naturelle.

Elle se rapporte plus précisément à un procédé de séparation d'un composé organique contenu dans une matière d'origine naturelle, qui comprend au moins une étape d'extraction liquide/liquide au moyen d'un système de solvants biphasique particulier.

L'invention se rapporte également à un procédé de purification de ce composé organique qui met en oeuvre le procédé de séparation qui vient d'être mentionné.

Les composés organiques que l'on cherche à séparer et, le cas échéant, à purifier sont notamment la pipérine et le cannabidiol.

### ÉTAT DE LA TECHNIQUE ANTÉRIEURE

La pipérine est un alcaloïde qui est contenu dans les plantes de la famille des Pipéracées et qui confère notamment le goût piquant au poivre noir (*piper nigrum*).

Le cannabidiol est, quant à lui, un composé organique de la famille des cannabinoïdes qui sont naturellement présents dans les plantes de la famille des Cannabinacées telle que la plante de cannabis, ou chanvre, au même titre que de nombreux autres composés organiques que sont les flavonoïdes, les stilbénoïdes, les terpènes et les alcaloïdes.

La pipérine et le cannabidiol, qui est également connu sous son abréviation CBD, sont des composés organiques particulièrement recherchés pour leurs propriétés thérapeutiques, la pipérine, notamment pour ses propriétés chimio-préventives, antioxydantes, anti-inflammatoires, antivirales, anti-Alzheimer et antidépressives, et le CBD, notamment pour ses propriétés non-psychotropes et ses effets pharmacologiques bénéfiques dans le traitement des maladies inflammatoires, du diabète, des cancers et de certaines maladies neurodégénératives.

La séparation de la pipérine ainsi que celle du CBD à partir de la matière d'origine naturelle, en l'espèce d'origine végétale, dans laquelle ces composés organiques sont contenus peuvent être réalisées par une extraction liquide/liquide qui permet de séparer le composé organique visé des autres composés et impuretés présents dans la matière végétale.

Ces séparations peuvent avantageusement être mises en oeuvre par une chromatographie de partage centrifuge (ou CPC) qui est fondée sur la différence de partage du soluté, ici le composé organique, entre deux phases liquides non miscibles d'un système de solvants biphasique, l'une des phases liquides formant la phase stationnaire, l'autre phase liquide formant la phase mobile.

Grâce à sa nature liquide, la séparation par CPC est entièrement gouvernée par la distribution, définie par le coefficient de partage Kd, du composé organique entre ces phases stationnaire et mobile. Par conséquent, la réussite de la séparation nécessite la recherche minutieuse d'un système de solvants biphasique permettant une séparation performante du composé organique d'intérêt.

Les systèmes de solvants biphasiques utilisés en CPC comprennent au moins deux solvants et, plus généralement, deux, trois ou quatre solvants.

Parmi les solvants les plus couramment utilisés en CPC, on peut citer les alcanes (heptane, hexane), les alcools (méthanol, éthanol, butanol), l'acétate d'éthyle, l'acétonitrile et l'eau.

Plus particulièrement, les systèmes biphasiques utilisés en CPC pour la séparation de la pipérine et celle du cannabidiol comprennent notamment de l'heptane, du méthanol et, le cas échéant, de l'acétate d'éthyle, qui sont des solvants toxiques, dans le sens où ils présentent des dangers non négligeables pour la santé humaine ainsi que pour l'environnement. De tels systèmes biphasiques utilisés pour séparer par CPC du cannabidiol sont notamment décrits dans les documents WO 2019/145552 A1, US 2021/230206 A1 et EP 3 449 992 A1.

D'autres systèmes de solvants biphasiques utilisés en CPC sont également décrits dans le document WO 2006/064105 A1 et la publication de T. Hammerschick et al. (" Isolation ofplastochromanol-8 from flaxseed oil by countercurrent séparation methods", Food Chemistry, 2022, vol.409, p.135345) pour la séparation de galanthamine et de plastochromanol-8, respectivement.

Il existe donc un besoin clairement exprimé de trouver une alternative aux systèmes de solvants biphasiques actuellement utilisés en CPC pour s'inscrire dans une démarche active de développement durable, et plus particulièrement dans une démarche dite de "chimie verte" qui vise à réduire, si ce n'est à supprimer, le recours aux solvants toxiques tels que ceux mentionnés ci-dessus.

Le but de la présente invention est, par conséquent, de pallier les inconvénients des procédés de l'art antérieur et, par conséquent, de proposer un procédé permettant de séparer par CPC, de manière performante et sélective, un composé organique d'intérêt tel que la pipérine ou le cannabidiol au moyen d'un système de solvants biphasique ne présentant pas la toxicité des systèmes biphasiques actuellement utilisés.

Un autre but de la présente invention est de proposer un procédé qui ne se limite pas uniquement à la séparation par CPC d'un composé organique d'intérêt, mais également et plus généralement à la séparation par extraction liquide/liquide, de ce composé organique d'intérêt présent dans une matière d'origine naturelle.

### EXPOSÉ DE L'INVENTION

Les buts précédemment énoncés ainsi que d'autres sont atteints, en premier lieu, par un procédé de séparation d'un composé organique contenu dans une matière d'origine naturelle du type précité, c'est-à-dire par un procédé qui comprend au moins une étape d'extraction liquide/liquide au moyen d'un système de solvants biphasique.

Selon l'invention, le système de solvants biphasique comprend de l'hexaméthyldisiloxane, de l'éthanol et de l'eau.

Les Inventeurs ont constaté que, de manière inattendue et surprenante, la mise en oeuvre d'un système biphasique comprenant de l'hexaméthyldisiloxane, de l'éthanol et de l'eau permet d'obtenir une séparation au moins aussi performante et sélective, si ce n'est plus performante et sélective, que celle obtenue avec un système biphasique actuel comprenant de l'hexane, ou de l'heptane, du méthanol et de l'eau et ce, sans les inconvénients liés à la nocivité du système biphasique actuel.

Cette séparation performante et sélective est obtenue par les substitutions respectives de l'hexaméthyldisiloxane et de l'éthanol à l'alcane et au méthanol, l'hexaméthyldisiloxane et l'éthanol présentant l'avantage d'être des solvants non toxiques, disponibles commercialement et d'un coût accessible.

Dans un mode de réalisation particulier du procédé de séparation selon l'invention, le système de solvants biphasique comprend :
- de 40 % vol à 60 % vol d'hexaméthyldisiloxane,
- de 25 % vol à 50 % vol d'éthanol, et
- jusqu'à 25 % vol d'eau.

Dans un mode de réalisation plus particulièrement avantageux, le système de solvants biphasique comprend :
- de 45 % vol à 55 % vol d'hexaméthyldisiloxane,
- de 30 % vol à 35 % vol d'éthanol, et
- de 15 % vol à 20 % vol d'eau.

Il est précisé que les intervalles de proportions volumiques qui viennent d'être mentionnés et qui sont précisés dans la suite de la présente demande sont à rapporter au volume total du système de solvants biphasique.

Il est également précisé que l'expression "comprend de ... à ...", qui vient d'être citée et qui est utilisée dans la présente demande, doit être comprise comme définissant non seulement les valeurs de l'intervalle, mais également les valeurs des bornes de cet intervalle.

Comme indiqué précédemment, le système de solvants biphasique mis en oeuvre dans le procédé de séparation selon l'invention comprend de l'hexaméthyldisiloxane, de l'éthanol et de l'eau. L'eau est, de préférence, de l'eau distillée.

Ce système biphasique peut ne comprendre que ces trois solvants mais peut également tout à fait comprendre un ou plusieurs autres solvants additionnels, comme on le verra par la suite.

Ainsi, selon une première variante du procédé de séparation selon l'invention, le système de solvants biphasique est constitué d'hexaméthyldisiloxane, d'éthanol et d'eau.

Une illustration de cette première variante particulière du système de solvants biphasique constitué d'hexaméthyldisiloxane, d'éthanol et d'eau est donnée dans les exemples qui sont détaillés ci-après en référence à la séparation du cannabidiol contenu dans une huile de chanvre.

Selon une deuxième variante du procédé de séparation selon l'invention, le système de solvants biphasique comprend, en plus de l'hexaméthyldisiloxane, de l'éthanol et de l'eau, au moins un solvant additionnel.

Ce solvant additionnel peut notamment être choisi parmi le 2-méthyltétrahydrofurane et l'isobutyrate d'éthyle.

Ainsi, dans un mode de réalisation particulier du procédé de séparation selon l'invention, le système de solvants biphasique comprend :
- de 10 % vol à 40 % vol d'hexaméthyldisiloxane,
- de 10 % vol à 40 % vol du solvant additionnel choisi parmi le 2-méthyltétrahydrofurane et l'isobutyrate d'éthyle,
- de 10 % vol à 40 % vol d'éthanol, et
- de 10 % vol à 40 % vol d'eau.

Dans un mode de réalisation plus particulièrement avantageux, le système de solvants biphasique comprend :
- de 20 % vol à 35 % vol d'hexaméthyldisiloxane,
- de 15 % vol à 30 % vol du solvant additionnel choisi parmi le 2-méthyltétrahydrofurane et l'isobutyrate d'éthyle,
- de 20 % vol à 35 % vol d'éthanol, et
- de 15 % vol à 30 % vol d'eau.

Selon une troisième variante du procédé de séparation selon l'invention, le système de solvants biphasique peut ne comprendre qu'un seul solvant additionnel, et notamment le solvant additionnel mentionné précédemment, en plus de l'hexaméthyldisiloxane, de l'éthanol et de l'eau.

En particulier, le système de solvants biphasique peut être constitué :
- d'hexaméthyldisiloxane, de 2-méthyltétrahydrofurane, d'éthanol et d'eau, ou
- d'hexaméthyldisiloxane, d'isobutyrate d'éthyle, d'éthanol et d'eau.

De manière avantageuse, le système de solvants biphasique est constitué d'hexaméthyldisiloxane, d'isobutyrate d'éthyle, d'éthanol et d'eau.

Des illustrations de cette troisième variante particulière, et notamment de cette troisième variante avantageuse du système de solvants biphasique constitué d'hexaméthyldisiloxane, d'isobutyrate d'éthyle, d'éthanol et d'eau, sont données dans les exemples qui sont détaillés ci-après en référence à la séparation de la pipérine contenue dans du poivre noir.

La matière d'origine naturelle à partir de laquelle le composé organique est séparé peut être choisie parmi les insectes, les champignons, les levures, les bactéries, les algues et microalgues, les mouts de fermentation et les plantes.

Lorsque cette matière d'origine naturelle est une matière végétale, elle peut être choisie parmi les plantes de la famille des Anacardiacées telles que le faux-poivrier ou Schinus molle, les plantes de la famille des Pipéracées telles que le poivre, et les plantes de la famille des Cannabinacées telles que le chanvre.

En particulier, le procédé de séparation selon l'invention permet de séparer, de manière particulièrement performante et sélective, la pipérine qui est un des composés organiques contenus dans le poivre ainsi que le cannabidiol contenu dans le chanvre parmi de nombreux autres composés organiques.

Dans un mode de réalisation particulier, le procédé de séparation selon l'invention peut comprendre, en outre, au moins une étape de transformation pour solubiliser la matière d'origine naturelle, ce ou ces étapes de transformation étant préalables à la mise en oeuvre de l'étape d'extraction liquide/liquide proprement dite.

Parmi les étapes de transformation susceptibles d'être mises en oeuvre sur la matière d'origine naturelle, on peut notamment citer :
- une étape de préparation de cette matière parmi lesquels la récolte, le séchage et/ou le broyage ;
- une étape d'extraction solide/liquide parmi lesquelles la macération, l'extraction sous CO₂ et/ou l'hydrodistillation ; et/ou
- une étape d'enrichissement ou de concentration parmi lesquelles une extraction liquide/liquide et/ou une distillation moléculaire.

Le procédé de séparation selon l'invention comprend au moins une étape d'extraction liquide/liquide au moyen du système de solvants biphasique décrit précédemment.

Cette étape d'extraction liquide/liquide peut être mise en oeuvre par toute technique connue dans ce domaine.

Plus avantageusement, et lorsque la matière d'origine naturelle est solubilisée, cette étape d'extraction liquide/liquide est mise en oeuvre par chromatographie à contrecourant (CCC) ou par chromatographie de partage centrifuge (CPC).

De préférence, cette étape d'extraction liquide/liquide est mise en oeuvre par chromatographie de partage centrifuge (CPC)

Ainsi, les extractions liquide-liquide qui sont conduites, à ce jour, au moyen d'un système biphasique comprenant de l'hexane, ou de l'heptane, du méthanol et de l'eau pour séparer un composé organique contenu dans une matière d'origine naturelle peuvent être avantageusement conduites au moyen d'un système biphasique comprenant de l'hexaméthyldisiloxane, de l'éthanol et de l'eau et ce, avec la mise en oeuvre de proportions volumiques respectives similaires, voire identiques.

De la même manière, les extractions liquide-liquide qui sont conduites, à ce jour, au moyen d'un système biphasique comprenant de l'hexane, ou de l'heptane, de l'acétate d'éthyle, du méthanol et de l'eau pour séparer un composé organique contenu dans une matière d'origine naturelle peuvent être avantageusement conduites au moyen d'un système biphasique comprenant soit de l'hexaméthyldisiloxane, du 2-méthyltétrahydrofurane, de l'éthanol et de l'eau, soit de l'hexaméthyldisiloxane, de l'isobutyrate d'éthyle, de l'éthanol et de l'eau et ce, comme précédemment indiqué, dans des proportions volumiques respectives similaires, voire identiques.

La présente invention se rapporte, en deuxième lieu, à un procédé de purification d'un composé organique contenu dans une matière d'origine naturelle.

Selon l'invention, ce procédé comprend les étapes (1) et (2) successives suivantes :
(1) une séparation du composé organique par un procédé de séparation tel que défini ci-dessus ; et
(2) une collecte de la phase liquide comprenant le composé organique telle qu'obtenue à l'issue de l'étape (1).

Dans ce procédé de purification selon l'invention, l'étape (1) de séparation est réalisée par le procédé de séparation tel que défini ci-avant, étant précisé que les caractéristiques avantageuses et préférentielles de ce procédé de séparation, telles que celles relatives à la matière d'origine naturelle, aux étapes de transformation de celle-ci, aux composés organiques, aux compositions des systèmes de solvants biphasiques ainsi qu'à l'étape d'extraction liquide/liquide proprement dite, peuvent être prises seules ou en combinaison.

Dans un mode de réalisation particulier, le procédé de purification selon l'invention, comprend, en outre, après l'étape (2) de collecte, une étape (3) de séparation du composé organique de la phase liquide collectée à l'étape (2) de manière à isoler le composé organique.

Cette étape (3) de séparation peut être mise en oeuvre par toute technique connue dans ce domaine. Elle peut notamment être mise en oeuvre par évaporation à sec, par précipitation ou par cristallisation.

### BRÈVE DESCRIPTION DES FIGURES

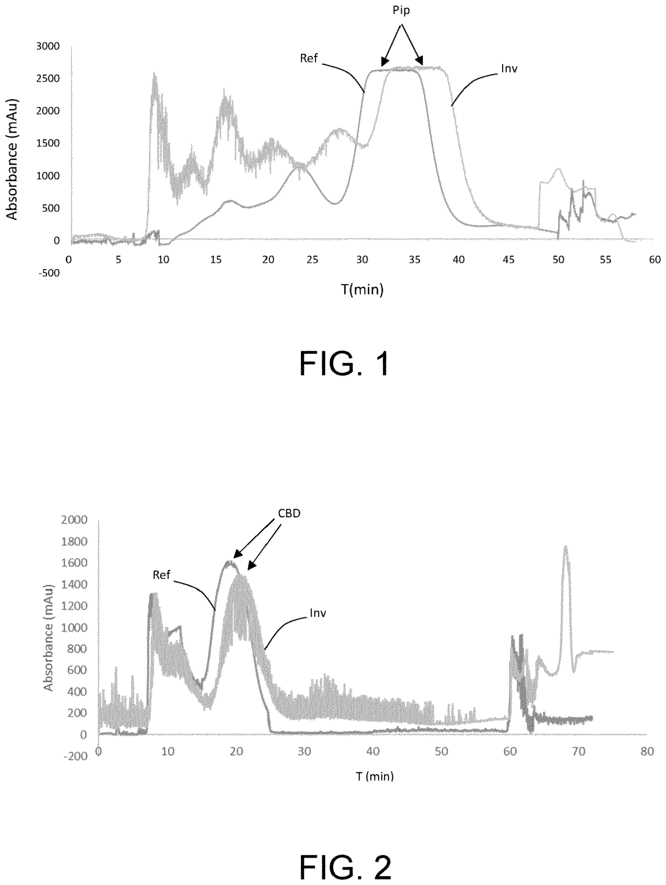

D'autres avantages, buts et caractéristiques particulières de la présente invention ressortiront de la description non limitative qui suit et qui se rapporte à des procédés de séparation et de purification faisant l'objet de la présente invention, en regard des figures 1 à 3 annexées, dans lesquelles :
- la figure 1 reproduit les chromatogrammes résultant de la séparation par une extraction liquide/liquide par CPC de pipérine conduite avec deux systèmes de solvants biphasiques distincts, l'un conventionnel (noté "Ref"), l'autre selon l'invention (noté "Inv") ;
- la figure 2 reproduit les chromatogrammes résultant de la séparation par une extraction liquide/liquide par CPC de cannabidiol conduite avec deux systèmes de solvants biphasiques distincts, l'un conventionnel (noté "Ref"), l'autre selon l'invention (noté "Inv") ; et
- la figure 3 reproduit les chromatogrammes résultant de la séparation par une extraction liquide/liquide par CPC de pipérine conduite avec deux systèmes de solvants biphasiques selon l'invention (notés "Inv-1" et "Inv-2").

### EXEMPLES

Les solvants formant les systèmes biphasiques mis en oeuvre dans les extractions liquide/liquide décrites ci-après sont les suivants :
- l'heptane, l'acétate d'éthyle (AcEt), le méthanol, l'éthanol absolu ainsi que l'eau distillée commercialisés par la société VWR International S.A.S (Rosny-sous-Bois, France) ;
- l'hexaméthyldisiloxane (HMDSO) et l'isobutyrate d'éthyle (EIB) commercialisés par la société Thermo Fisher Scientific (Heysham, Royaume-Uni) ; et
- le 2-méthyltétrahydrofurane (2-MeTHF) commercialisé par la société Sigma-Aldrich (Saint-Quentin-Fallavier, France).

La pipérine a été purifiée à partir d'un échantillon d'extrait brut de poivre noir. Pour l'obtention de celui-ci, environ 500 g de poivre noir moulu fourni par la société Samia (La Courneuve, France) ont été extraits avec 2 L de dichlorométhane. Après concentration à sec, on a obtenu un échantillon de 33 g d'extrait brut de poivre noir.

Le cannabidiol (CBD) a été purifié à partir d'un échantillon d'huile de chanvre commercialisée par la société Cibdol (Bâle, Suisse). Il est précisé que le flacon contenant cette huile de chanvre indiquait que cette huile comprenait une proportion pondérale de 2 % de CBD.

Ces échantillons d'extrait brut de poivre noir et d'huile de cannabinoïdes ont été conservés à 4 °C avant ainsi qu'entre les tests.

Les tests de purification de la pipérine, d'une part, et du cannabidiol, d'autre part, ont été conduits au moyen d'un dispositif de chromatographie de partage centrifuge (CPC) doté d'une colonne de 250 mL de capacité et d'un dispositif de chromatographie en phase liquide (PLC), dispositifs qui sont commercialisés par la société Gilson Purification SAS (Saint-Avé, France) sous les dénominations commerciales respectives "Verity CPC 250" et "PLC 2250". Le dispositif de PLC est équipé de vannes d'injection et de rinçage, d'une pompe quaternaire, d'un collecteur de fractions et d'un détecteur UV/vis balayant les longueurs d'onde de 200 nm à 600 nm. La séparation et les données ont été traitées avec le logiciel "Gilson Glider Prep.".

Différents systèmes de solvants biphasiques ont été préparés en mélangeant les volumes correspondants de solvants.

Pour la purification de la pipérine :
- un test "de référence" a été mis en oeuvre avec un système de solvants biphasique conventionnel comprenant de l'heptane, de l'acétate d'éthyle, du méthanol et de l'eau distillée dans les proportions volumiques respectives de 6/5/6/5, et
- un test selon l'invention a été mis en oeuvre avec un système de solvants biphasique comprenant de l'hexaméthyldisiloxane, de l'isobutyrate d'éthyle, de l'éthanol et de l'eau distillée dans les proportions volumiques respectives de 3/2/3/2.

Pour la purification du CBD :
- un test "de référence" a été mis en oeuvre avec un système de solvants biphasique conventionnel comprenant de l'heptane, du méthanol et de l'eau distillée dans les proportions volumiques respectives de 5/4/1, et
- un test selon l'invention a été mis en oeuvre avec un système de solvants biphasique comprenant de l'hexaméthyldisiloxane, de l'éthanol et de l'eau distillée dans les proportions volumiques respectives de 5/3,5/1,5.

Les séparations par CPC ont été réalisées en utilisant l'élution-extrusion en mode ascendant pour la purification de la pipérine et en mode descendant pour la purification du CBD. Les deux séparations ont été effectuées à une vitesse de rotation de 2000 tr/min avec un débit maintenu à 8 mL/min pendant l'élution et augmenté à 30 mL/min pendant l'extrusion.

Les paramètres optimaux des séparations de la pipérine, d'une part, et du cannabidiol, d'autre part, sont reportés dans le Tableau 1 ci-après.

Pour l'injection, les échantillons ont d'abord été dilués dans 2 mL de phase supérieure et 2 mL de phase inférieure. Les solutions ont ensuite été filtrées (0,45 µm) avant l'injection. Les échantillons ont été injectés à l'aide d'une boucle d'injection de 5 mL pré-remplie avec la phase mobile.

**Tableau 1**

| | Pipérine | Cannabidiol (CBD) |
|---|---|---|
| Mode de séparation | ascendant | descendant |
| Volume de la colonne | 250 mL | |
| Débit | 8 mL/min | |
| Vitesse de rotation | 2000 tr/min | |
| Boucle d'injection | 5 mL | |
| Préparation de l'échantillon | 300 mg dans 4 mL de phases supérieure et inférieure (1/1, v/v) | 1 g dans 4 mL de phases supérieure et inférieure (1/1, v/v) |
| Détection | 250 nm, 284 nm, 300 nm et Scan 200-600 nm | 280 nm, 305 nm et Scan 200-600 nm |

En référence aux chromatogrammes de la figure 1, on observe que le système de solvants biphasique mis en oeuvre dans le cadre de la présente invention permet d'obtenir une séparation de la pipérine qui est performante et comparable, notamment en termes de sélectivité et de temps de séparation, à celle que l'on obtient avec le système de solvants biphasique conventionnel comprenant un alcane, en l'espèce de l'heptane. Les pics correspondants à chacune des fractions de pipérine séparée sont repérés par la flèche notée "Pip".

La fraction de pipérine séparée et collectée à l'issue de l'extraction liquide/liquide, qui a été mise en oeuvre dans le procédé selon l'invention, a été analysée par chromatographie sur couche mince (CCM). Cette analyse CCM montre que la pipérine, qui a été séparée et purifiée au moyen du système de solvants biphasique particulier mis en oeuvre dans les procédés selon l'invention, est pure.

En référence aux chromatogrammes de la figure 2 et, en particulier aux pics correspondants à chacune des fractions de CBD séparé et repérés par la flèche notée "CBD", on observe que l'on obtient également une séparation performante et sélective du cannabidiol (CBD) au moyen du système de solvants biphasique mis en oeuvre dans le cadre de la présente invention.

Une analyse CCM a également permis de confirmer la pureté du CBD séparé et purifié au moyen du système de solvants biphasique particulier mis en oeuvre dans les procédés selon l'invention.

Un bilan massique a, en outre, montré que la fraction de CBD obtenu par le procédé de purification selon l'invention représentait 1,8 % contre 1,5 % pour la fraction de CBD obtenu par le procédé mettant en oeuvre le système de solvants biphasique conventionnel.

Deux autres tests de séparation de la pipérine ont également été réalisés avec un système de solvants biphasique comprenant de l'hexaméthyldisiloxane, de l'isobutyrate d'éthyle, de l'éthanol et de l'eau distillée dans des proportions volumiques respectives de 6/5/6/5 ("Inv-1") et de 1/2/1/2 ("Inv-2") et ce, dans les mêmes conditions opératoires que celles mentionnées dans le Tableau 1 ci-dessus.

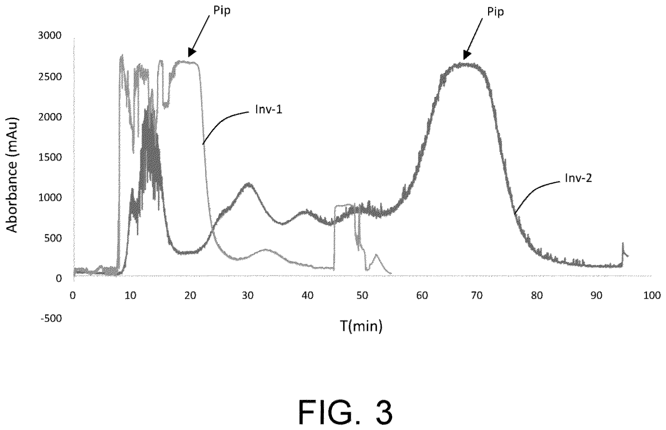

En référence aux chromatogrammes correspondants de la figure 3 et, en particulier, aux pics relatifs à chacune des fractions de pipérine séparée et repérés par la flèche notée "Pip", on observe que les systèmes de solvants biphasiques mis en oeuvre dans le cadre de la présente invention permettent d'obtenir une séparation de la pipérine particulièrement sélective et ce, pour des proportions volumiques différentes et s'inscrivant dans de larges intervalles.

Ces conclusions sur les performances de séparation et, partant, de purification sont également transposables à d'autres systèmes de solvants biphasiques dont les compositions sont indiquées ci-après :
* d'une part, pour la séparation de la pipérine :
   - un système de solvants biphasique comprenant HMDSO, EIB, de l'éthanol et de l'eau distillée dans les proportions volumiques respectives de 2/1/2/1,
   - un système de solvants biphasique comprenant HMDSO, 2Me-THF, de l'éthanol et de l'eau distillée dans les proportions volumiques respectives de 5/6/5/6 ; et
* d'autre part, pour la séparation du CBD :
   - des systèmes de solvants biphasiques comprenant HMDSO, de l'éthanol et de l'eau distillée dans les proportions volumiques respectives de 5/4,5/0,5, 5/4/1 et 5/3/2.

## Revendications

1. Procédé de séparation d'un composé organique contenu dans une matière d'origine naturelle, ledit procédé comprenant au moins une étape d'extraction liquide/liquide au moyen d'un système de solvants biphasique, **caractérisé en ce que** le système de solvants biphasique comprend de l'hexaméthyldisiloxane, de l'éthanol et de l'eau, de préférence de l'eau distillée.

2. Procédé selon la revendication 1, dans lequel le composé organique est choisi parmi la pipérine et le cannabidiol.

3. Procédé selon la revendication 1 ou 2, dans lequel le système de solvants biphasique comprend, les proportions volumiques étant indiquées par rapport au volume total du système de solvants biphasique :
- de 40 % vol à 60 % vol et, avantageusement, 45 % vol à 55 % vol d'hexaméthyldisiloxane,
- de 25 % vol à 50 % vol et, avantageusement, de 30 % vol à 35 % vol d'éthanol, et
- jusqu'à 25 % vol et, avantageusement, de 15 % vol à 20 % vol d'eau.

4. Procédé selon l'une quelconque des revendications 1 à 3, dans lequel le système de solvants biphasique est constitué d'hexaméthyldisiloxane, d'éthanol et d'eau.

5. Procédé selon la revendication 1 ou 2, dans lequel le système de solvants biphasique comprend, en outre, un solvant additionnel choisi parmi le 2-méthyltétrahydrofurane et l'isobutyrate d'éthyle.

6. Procédé selon la revendication 5, dans lequel le système de solvants biphasique comprend, les proportions volumiques étant indiquées par rapport au volume total du système de solvants biphasique :
- de 10 % vol à 40 % vol et, avantageusement, de 20 % vol à 35 % vol d'hexaméthyldisiloxane,
- de 10 % vol à 40 % vol et, avantageusement, de 15 % vol à 30 % vol du solvant additionnel,
- de 10 % vol à 40 % vol et, avantageusement, de 20 % vol à 35 % vol d'éthanol, et
- de 10 % vol à 40 % vol et, avantageusement, de 15 % vol à 30 % vol d'eau.

7. Procédé selon la revendication 5 ou 6, dans lequel le système de solvants biphasique est constitué d'hexaméthyldisiloxane, d'éthanol, d'eau et d'un solvant additionnel choisi parmi le 2-méthyltétrahydrofurane et l'isobutyrate d'éthyle, le système de solvants biphasique étant, avantageusement, constitué d'hexaméthyldisiloxane, d'isobutyrate d'éthyle, d'éthanol et d'eau.

8. Procédé selon l'une quelconque des revendications 1 à 7, comprenant, en outre, au moins une étape de transformation pour solubiliser la matière d'origine naturelle, ce ou ces étapes de transformation étant préalables à la mise en oeuvre de l'étape d'extraction liquide/liquide.

9. Procédé selon la revendication 1 à 7, dans lequel l'étape d'extraction liquide/liquide est mise en oeuvre par chromatographie à contrecourant ou par chromatographie de partage centrifuge et, de préférence, par chromatographie de partage centrifuge.

10. Procédé de purification d'un composé organique contenu dans une matière végétale, **caractérisé en ce qu'**il comprend les étapes (1) et (2) successives suivantes :
(1) une séparation du composé organique par un procédé de séparation selon l'une quelconque des revendications 1 à 9 ; et
(2) une collecte de la phase liquide comprenant le composé organique telle qu'obtenue à l'issue de l'étape (1).

11. Procédé selon la revendication 10, comprenant, en outre, après l'étape (2) de collecte, une étape (3) de séparation du composé organique de la phase liquide collectée à l'étape (2), cette étape de séparation pouvant notamment être mise en oeuvre par évaporation à sec, par précipitation ou par cristallisation.

12. Procédé de séparation selon l'une quelconque des revendications 1 à 9 ou procédé de purification selon la revendication 10 ou 11, dans lequel la matière d'origine naturelle est choisie parmi les insectes, les champignons, les levures, les bactéries, les algues et microalgues, les mouts de fermentation et les plantes, telles que les plantes de la famille des Anacardiacées, notamment le faux-poivrier ou Schinus molle, de la famille des Pipéracées, notamment le poivre, et de la famille des Cannabinacées, notamment le chanvre.
